# EUROPÄISCHE PATENTSCHRIFT

(11) **EP 2 695 933 B1**
(45) Veröffentlichungstag und Bekanntmachung des Hinweises auf die Patenterteilung: **18.09.2019**
(21) Anmeldenummer: 13191654.6
(22) Anmeldetag: 09.04.2008
(51) Int. Cl.: C12M 1/22

(54) **Behältnis zur Aufnahme von Nährmedien**
Receptacle for accepting nutrient media
Contenant de reception de milieux nutritifs

(30) Priorität: 24.05.2007 DE 102007024620; 11.06.2007 DE 102007027273
(43) Veröffentlichungstag der Anmeldung: 12.02.2014
(62) Teilanmeldung aus: 08734471.9
(73) Patentinhaber: Merck Patent GmbH, 64293 Darmstadt (DE)
(72) Erfinder: Müller, Rolf, 69221 Dossenheim (DE)
(74) Vertreter: Patent- und Rechtsanwälte Ullrich & Naumann

(56) Entgegenhaltungen:
- EP-A- 0 171 174
- EP-A- 0 848 057
- EP-A- 1 528 100
- DE-A1- 3 128 542
- DE-A1- 3 137 495
- DE-A1- 4 301 882
- DE-C1- 19 608 009
- DE-T2- 60 009 564
- JP-A- 2004 337 079
- US-A- 3 055 808
- US-A- 3 158 553
- US-A- 5 021 351
- US-A- 5 605 836
- US-A1- 2001 024 821
- US-A1- 2004 224 382
- US-A1- 2006 240 549
- US-B2- 6 756 225

## Beschreibung

Die Erfindung betrifft ein Behältnis zur Aufnahme von Nährmedien, insbesondere zur Kultivierung von Mikroorganismen, Zellkulturen, Bakterien, mit einer Schale und einem die Schale schließenden Deckel, wobei die Schale einen in Form einer Kreisfläche ausgebildeten Schalenboden und eine vom Schalenboden abragende, kreisringförmige Schalenwand umfasst und wobei der Deckel einen in Form einer Kreisfläche ausgebildeten Deckelboden und eine vom Deckelboden abragende, kreisringförmige Deckelwand umfasst, wobei die Deckelwand einen größeren Innendurchmesser als der Außendurchmesser der Schalenwand hat oder die Schalenwand einen größeren Innendurchmesser als der Außendurchmesser der Deckelwand hat, so dass zum Schließen der Schale die Deckelwand über die Schalenwand oder die Schalenwand über die Deckelwand stülpbar ist, wobei der Schale und dem Deckel Eingriffsmittel zugeordnet sind, die bei gegenseitigem Eingriff ein unbeabsichtigtes Lösen von Schale und Deckel verhindern und wobei die Eingriffsmittel der Schale klammerartig oder flanschartig zum Um- oder Eingreifen der Eingriffsmittel des Deckels ausgeführt sind, und wobei sich die Flansche von einem Randbereich des Schalenbodens aus orthogonal erstrecken und eine für das Halten oder die Klemmwirkung verantwortliche Schulter aufweisen.

Die Schale und der Deckel umfassen jeweils einen in Form einer Kreisfläche ausgebildeten Boden (Schalenboden und Deckelboden) und eine vom Boden abragende, kreisringförmige Wand (Schalenwand und Deckelwand). Eine der Wände, vorzugsweise die Deckelwand, hat einen zumindest geringfügig größeren Innendurchmesser als der Außendurchmesser der anderen Wand, vorzugsweise der Schalenwand, so dass zum Schließen der Schale die eine Wand über die andere (oder umgekehrt) stülpbar ist.

Behältnisse der in Rede stehenden Art sind unter der Bezeichnung "Petrischale" bekannt. Darunter versteht man eine flache, runde, meist durchsichtige Glas- oder Kunststoffschale mit übergreifendem Deckel. Eine solche Schale kommt üblicherweise in der Biologie oder Chemie zum Einsatz. Sie dient zur Kultivierung von Mikroorganismen und wird zum Anlegen von Zellkulturen genutzt.

In Bezug auf das gattungsbildende Behältnis sei lediglich beispielhaft auf die DE 44 06 725 A1 verwiesen, aus der ein Behältnis im Sinne einer Petrischale bekannt ist. Ein Deckel dient zum Verschluss des Behältnisses. Regelmäßig wird der Deckel über die Schale bzw. über die Schalenwand gestülpt. Die Handhabung eines insoweit geschlossenen Behältnisses ist jedoch problematisch, da beim Ergreifen des Deckels ein Lösen von Schale und Deckel nicht zu vermeiden ist. Verunreinigungen gelangen so ins Innere des Behältnisses oder angelegte Kulturen können nach außerhalb des Behältnisses gelangen. Es besteht Kontaminationsgefahr.

Die Dokumente US 2006/240549 A1, EP 0 171 174 A, JP 2004 337079 A, DE 31 37 495 A1, US 5 021 351 A und US 6 756 225 B2 zeigen jeweils unterschiedliche Ausgestaltungen von Behältnisses zur Aufnahme von Nährmedien.

Die DE 43 01 882 A1 betrifft einen Kulturbehälter zum Züchten von Mikroorganismen. Der Kulturbehälter weist ein Verteilelement zum Verteilen von einer Nährbodenschicht und eine Umstelleinrichtung zum Umstellen des Deckels zwischen einer Aufbringeinstellung und einer Verteilstellung auf.

Weiterhin zeigt die EP 1 528 100 A eine Schale 20 und einen die Schale 20 schließenden Deckel 30, wobei die Schale 20 und der Deckel 30 jeweils einen in Form einer Kreisfläche ausgebildeten Boden und eine vom Boden abragende, kreisförmige Wand umfassen. Die Wand des Deckels 30 hat einen größeren Innendurchmesser als der Außendurchmesser der Wand der Schale 20, so dass der Deckel 30 über die Schale 20 stülpbar ist. Der Schale 20 und dem Deckel 30 sind des Weiteren Eingriffsmittel 12, 36 zugeordnet, die bei gegenseitigem Eingriff ein unbeabsichtigtes Lösen des Deckels 30 von der Schale 20 verhindern. Dabei sind die Eingriffsmittel 12 der Schale 20 derart ausgebildet, dass sie die Eingriffsmittel 36 des Deckels 30 umgreifen.

Die US 3 158 553 A zeigt eine Petrischale, die als geschlossene Einheit oder als offene Einheit verwendbar ist. Die Schale 10 weist Nasen 14 auf, die sich seitlich von dem Rand der Schale 10 weg erstrecken. Auf die Nasen 14 ist ein keilförmiger Bereich 15 des Deckels 12 auflegbar. Je nachdem wie der Deckel 12 gegenüber der Schale 10 gedreht ist, liegen die Nasen in einer Ausnehmung 17 des keilförmigen Elements 15 oder auf einer keilförmigen Oberfläche 16 auf, woraus sich die geschlossene bzw. teilweise geöffnete Stellung ergibt. Der Deckel 12 ist dabei lediglich auf die Schale 10 aufgelegt.

Aus der Praxis ist bereits bekannt, durch eine geeignete Ausgestaltung und Dimensionierung von Schale und Deckel zwischen der Schale und dem Deckel eine Klemmwirkung hervorzurufen. Wollte man dabei eine sichere Verbindung zwischen der Schale und dem Deckel generieren, müsste man eine Art Presspassung realisieren, die ein Lösen des Deckels von der Schale erschwert, wenn nicht sogar unmöglich macht. Folglich ist eine solche Lösung zum sicheren Verschleißen und Öffnen des Behältnisses nur bedingt geeignet.

Der vorliegenden Erfindung liegt nun die Aufgabe zugrunde, ein Behältnis zur Aufnahme von Nährmedien, insbesondere zum Kultivieren von Mikroorganismen, Zellkulturen bzw. Organismen, derart auszugestalten und weiterzubilden, dass ein unbeabsichtigtes Lösen des Deckels von der Schale weitestgehend vermieden ist.

Erfindungsgemäß ist die voranstehende Aufgabe durch die Merkmale des Patentanspruches 1 gelöst. Danach ist das hier in Rede stehende Behältnis dadurch gekennzeichnet, dass die Eingriffsmittel der Schale, in Umfangsrichtung der Schale gesehen hälftig von beiden Seiten her, unterschiedlich hohe Keilflächen oder Erhöhungen haben, so dass die Eingriffsmittel des Deckels in Umfangsrichtung des Deckels gesehen von beiden Seiten her mit unterschiedlicher Klemm- und Dichtwirkung in die Eingriffsmittel der Schale, nämlich in den Bereich unter die Schulter, einschiebbar sind.

In erfindungsgemäßer Weise ist erkannt worden, dass die aus der Praxis bekannte Klemmwirkung zwischen Schale und Deckel einerseits unzureichend ist und andererseits die Gefahr in sich birgt, dass sich der Deckel von der Schale aufgrund einer besonders starken Klemmwirkung nicht lösen lässt. So ist man in erfindungsgemäßer Weise von einer konventionellen Verbindung zwischen Schale und Deckel aufgrund einer Klemmwirkung, nämlich aufgrund einer Presspassung, abgegangen, und hat eine Verbindung durch Eingriffsmittel gewählt, wobei diese Eingriffsmittel sowohl der Schale als auch dem Deckel zugeordnet sind. Die Eingriffsmittel sind derart ausgebildet bzw. konstruiert, dass bei gegenseitigem Eingriff der Eingriffsmittel ein unbeabsichtigtes Lösen des Deckels von der Schale wirksam verhindert ist. Bringt man die Eingriffsmittel außer Eingriff, lässt sich der Deckel mühelos von der Schale entfernen.

In Bezug auf eine konstruktiv einfache Ausgestaltung des Behältnisses ist es von Vorteil, wenn die Eingriffsmittel integrale Bestandteile der Schale einerseits und des Deckels andererseits sind. Dabei können die Eingriffsmittel der Deckelwand und der Schalenwand zugeordnet sein. Auch ist es denkbar, dass die Eingriffsmittel einerseits der Deckelwand und andererseits einem umlaufenden äußeren Randbereich der Schale zugeordnet sind. Wesentlich ist dabei lediglich, dass sowohl der Schale als auch dem Deckel Eingriffsmittel zugeordnet sind, die beim Schließen der Schale durch den Deckel in gegenseitigen Eingriff bringbar sind, so dass ein unbeabsichtigtes Lösen des Deckels von der Schale ausgeschlossen ist.

In weiter vorteilhafter Weise dient der äußere Randbereich der Schale, der aufgrund der Anordnung bzw. Ausbildung der Schalenwand außerhalb der Schale liegt, zur Anlage für die Deckelwand, und zwar dann, wenn die Deckelwand über die Schalenwand greift.

Entsprechend ist es von Vorteil, wenn die Schalenwand vom äußeren Randbereich nach innen versetzt ausgebildet ist und wenn der außerhalb der Schalenwand liegende Randbereich, vorzugsweise ganz außen, die Eingriffsmittel trägt. An dieser Stelle sei angemerkt, dass es hier ausschließlich um den gegenseitigen Eingriff der Eingriffsmittel geht, die sowohl der Schale als auch dem Deckel zugeordnet sind. Alternativ ist eine Ausgestaltung von Schale und Deckel der Gestalt möglich, dass die Schalenwand die Deckelwand übergreift, so dass der Deckel in die Schale, d. h. in den Bereich innerhalb der Schalenwand, einsetzbar ist. Auch im Rahmen einer solchen Ausgestaltung ist ein gegenseitiges in Eingriff bringen der Eingriffsmittel möglich, so dass auch insoweit die erfindungsgemäße Lehre anwendbar ist.

Lediglich stellvertretend für alle denkbaren Ausgestaltungsvarianten sei nachfolgend ausschließlich die Rede davon, dass der Deckel die Schale bzw. die Schalenwand übergreift, wenngleich auch eine andere Realisierung, entsprechend den voranstehenden Ausführungen, denkbar ist

Im Konkreten können die Eingriffsmittel des Deckels mindestens eine oder mehrere Rastposition umfassen, wobei die Rastposition so zu verstehen ist, dass die Eingriffsmittel des Deckels - symmetrisch oder unsymmetrisch - Reduktionen im Querschnitt, Hinterschneidungen, Materialverjüngung, etc. aufweisen, die gegenüber den Eingriffsmitteln der Schale die Rastposition bilden bzw. definieren.

Die Eingriffsmittel der Schale sind entsprechend der Ausgestaltung der Eingriffsmittel des Deckels ausgebildet, nämlich um die Eingriffsmittel des Deckels formschlüssig und/oder kraftschlüssig aufnehmen zu können. Dazu sind die Eingriffsmittel der Schale klammerartig oder flanschartig zum Um- oder Eingreifen der Eingriffsmittel des Deckels ausgeführt sind. Dabei ist es von weiterem Vorteil, wenn die Eingriffsmittel der Schale mindestens eine konstruktive Maßnahme zum klemmenden Eingriff der Eingriffsmittel des Deckels haben. Dabei kann es sich um eine Keilfläche oder um eine sonstige Erhöhung zum klemmenden Eingriff handeln.

Von ganz besonderem Vorteil ist eine weiterreichende Ausgestaltung der erfindungsgemäßen Lehre dahingehend, dass die Eingriffsmittel der Schale derart ausgebildet sind, dass die Eingriffsmittel des Deckels durch Drehen des Deckels gegenüber der Schale in Eingriff bringbar sind. Dabei ist es denkbar, dass - zusätzlich - zwischen der Schale und dem Deckel Dichtmittel wirken, bspw. ein auf den Randbereich der Schale aufgelegter Dichtring oder dergleichen.

Von ganz besonderem Vorteil ist, wenn die Eingriffsmittel des Deckels, in etwa hälftig von beiden Seiten her mit unterschiedlicher Klemm- und Dichtwirkung einschiebbar sind, unterschiedlich hohe und unterschiedlich geneigte Keilflächen haben, so dass die Eingriffsmittel des Deckels von beiden Seiten her mit unterschiedlicher Klemm- und Dichtwirkung einschiebbar sind. Im Rahmen einer solchen Ausgestaltung ist es möglich, den Deckel bzw. die Eingriffsmittel des Deckels jeweils links oder rechts von den Eingriffsmitteln der Schale zu positionieren und durch Drehen gegenüber der Schale die Eingriffsmittel des Deckels in die Eingriffsmittel der Schale - von links oder von rechts her - einzuschieben. Aufgrund der unterschiedlichen Keilflächen oder unterschiedlicher Erhöhung entsteht von der einen Seite her ein fester, abdichtender Verschluss der Schale und von der anderen Seite her Verschluss mit definiertem Schlitz bzw. Spalt zwischen der Schale und dem Deckel zum Gasaustausch. Somit lassen sich im Behältnis unterschiedliche Bedingungen für die jeweiligen Kulturen schaffen.

In Bezug auf ein sicheres Schließen der Schale ist es erforderlich, dass mindestens zwei vorzugsweise äquidistant angeordnete Eingriffsmittel vorgesehen sind. Eine kippsichere Anordnung des Deckels gegenüber der Schale und somit ein sicheres Schließen wird durch die Vorkehrung von drei äquidistant angeordneten Eingriffsmitteln bewerkstelligt, so dass der Deckel entlang seines Umfanges gleichmäßig zur Schale anordenbar ist.

In weiter vorteilhafter Weise lässt sich die Verbindung bzw. Positionierung des Deckels zur Schale dadurch begünstigen, dass die Schalenwand auf der Außenseite und/oder die Deckelwand auf der Innenseite Abstandhalter zur Beabstandung und ggf. zur Klemmung zwischen Deckelwand und Schalewand hat, so dass eine Belüftung der Schale dann jedenfalls gewährleistet ist, wenn sich der Deckel in der offenen, d. h. einen Spalt gegenüber der Schale bildenden Position befindet. Die geschlossene Position, ohne Vorkehrung eines Spalts zwischen der Schale und dem Deckel, wird dann erreicht, wenn die Deckelwand komplett an der Schale bzw. an den Randbereich der Schale gedrückt wird, nämlich durch entsprechendes Zusammenwirken zwischen den Eingriffsmitteln der Schale und des Deckels.

Im Konkreten können die Abstandhalter als sich vom Boden orthogonal erstreckende Stege ausgeführt sein. Entlang des Umfangs der Schalenwand lassen sich mehrere Abstandhalter entsprechend den voranstehenden Ausführungen anbringen, wobei es von ganz besonderem Vorteil ist, wenn gegenüber eines jeden Eingriffsmittels auch ein Abstandhalter angeordnet ist, so dass die Deckelwand dazwischen, d. h. im Bereich zwischen einem Eingriffsmittel und einem Abstandhalter, eingeführt bzw. eingesteckt werden kann. Diese Maßnahme begünstigt ein sicheres Positionieren des Deckels gegenüber der Schale.

Die Abstandhalter sind vorzugsweise äquidistant in der Schalenwand und/oder in der Deckelwand ausgebildet, wobei die Vorkehrung hinreichend vieler Abstandhalter eine gleichmäßige Beabstandung zwischen der Deckelwand und Schalenwand gewährleistet.

Die Handhabung des erfindungsgemäßen Behältnisses wird dadurch begünstigt, dass auf der der Schalenwand abgewanden Seite des Schalenbodens eine vorzugsweise am äußeren Ende des Randbereichs in entgegengesetzte Richtung abragende Basis in Form einer kreisringförmigen Wand ausgebildet ist, die zum Positionieren und ggf. Stapeln der Schale dient. Diese Wand bildet eine Art Podest für die eigentliche Schale und vereinfacht nicht nur die Handhabung sondern auch die Lagerung im leeren oder gefüllten Zustand.

In weiter vorteilhafter Weise ist es denkbar, dass sowohl die Schale als auch der Deckel, vorzugsweise mit entsprechender Zuordnung, mit einer vorzugsweise maschinenlesbaren Codierung (z. B. in Form eines Data Matrix Codes) versehen sind. Mit einer solchen Codierung lassen sich die angelegten Kulturen eindeutig zuordnen bzw. identifizieren.

Schließlich sei angemerkt, dass die Schale und der Deckel aus Glas oder aus vorzugsweise durchsichtigem Kunststoff hergestellt sein können. Im Falle einer zu bevorzugenden Ausgestaltung aus Kunststoff ist es denkbar, dass die Schale und der Deckel spritzgusstechnisch hergestellt sind. Eine spritzgusstechnische Fertigung hat den enormen Vorteil, dass sich sämtliche Bestandteile sowohl des Deckels als auch der Schale als integrale Bestandteile ausbilden lassen, was die Herstellkosten ganz erheblich reduziert.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Draufsicht ein Ausführungsbeispiel einer Schale eines erfindungsgemäßen Behältnisses,
- Fig. 2a: den Gegenstand aus Fig. 1, teilweise und vergrößert, im Schnitt entlang der Linie A-A,
- Fig. 2b: den Gegenstand aus Fig. 1 im Schnitt entlang der Linie C-C,
- Fig. 3a: in einer schematischen Draufsicht das Behältnis mit Schale und Deckel, wobei sich der Deckel in der offenen Position befindet,
- Fig. 3b: den Gegenstand aus Fig. 3a im Schnitt entlang der Linie D-D,
- Fig. 4a: in einer schematischen Draufsicht das Behältnis mit Schale und Deckel, wobei sich der Deckel in der geschlossenen Position befindet,
- Fig. 4b: den Gegenstand aus Fig. 4a im Schnitt entlang der Linie E-E,
- Fig. 5a: in einer schematischen Draufsicht den Deckel des erfindungsgemäßen Behältnisses und
- Fig. 5b: den Gegenstand aus Fig. 5a im Schnitt entlang der Linie B-B.

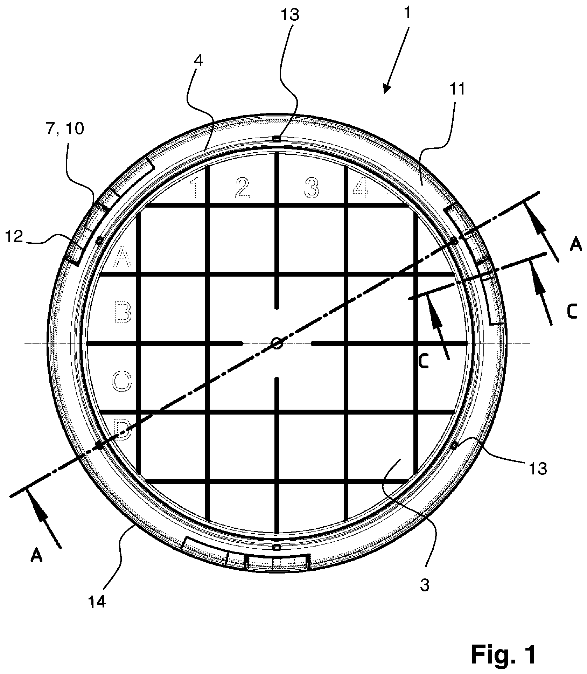

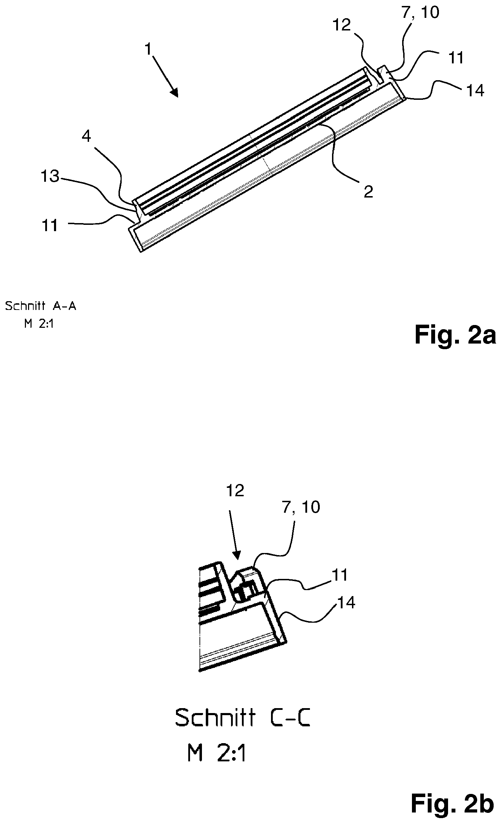

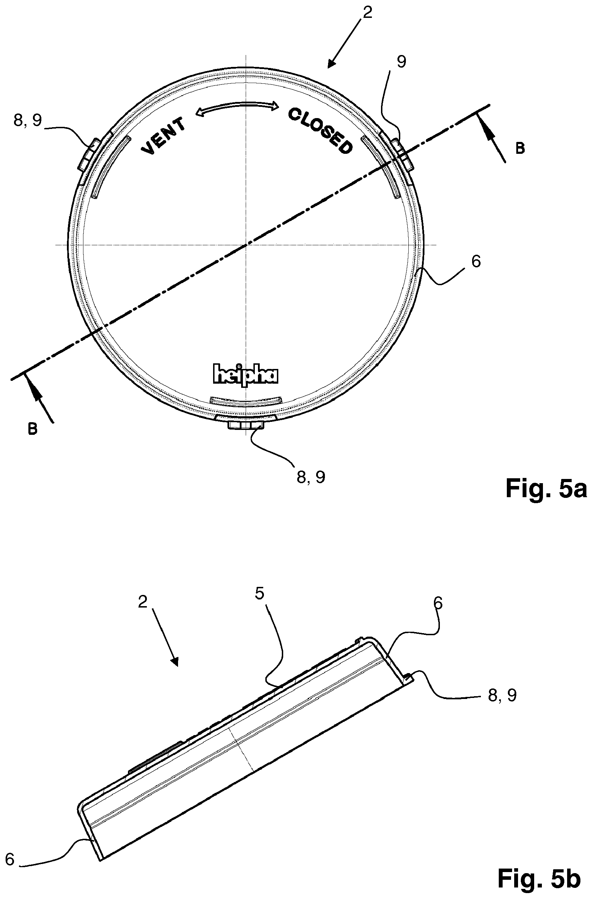

Die Fig. 1 bis 5 zeigen in schematischen Ansichten ein Ausführungsbeispiel eines erfindungsgemäßen Behältnisses zur Aufnahme von Nährmedien, insbesondere zur Aufnahme von Zellkulturen, Bakterien, etc. jedweder Art. Im Konkreten handelt es sich dabei um besondere Ausbildungen einer Petrischale.

Das Behältnis umfasst eine Schale 1 und einen Deckel 2. Der Deckel 2 dient zum Schließen der Schale 1.

Die Schale 1 umfasst einen in Form einer Kreisfläche ausgebildeten Schalenboden 3 und eine vom Schalenboden 3 abragende, kreisringförmige Schalenwand 4.

Der Deckel 2 umfasst entsprechend einen Deckelboden 5 und eine Deckelwand 6.

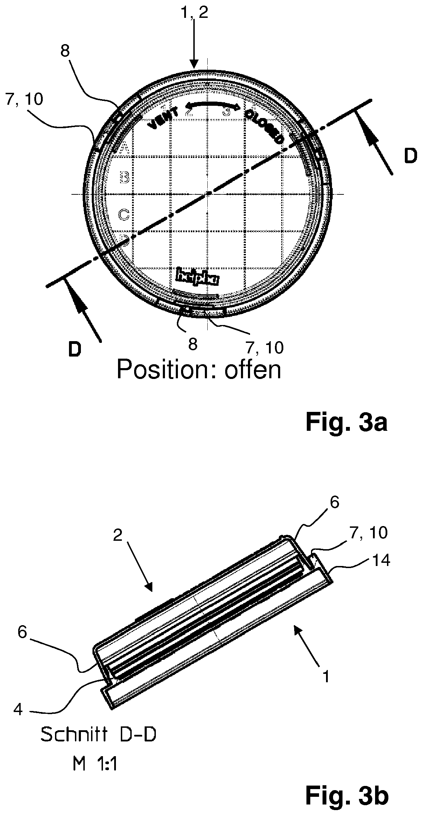

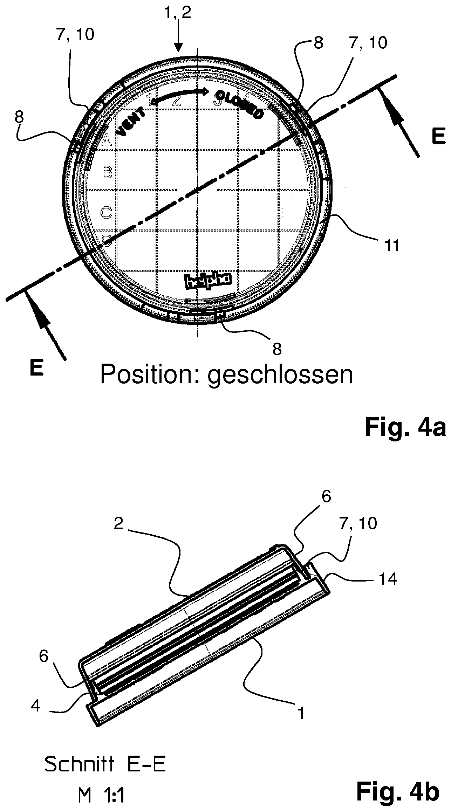

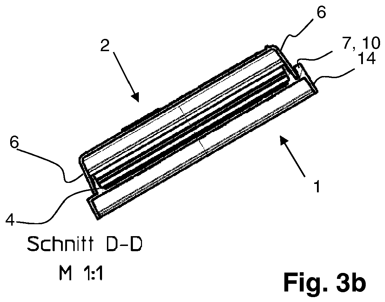

Die Fig. 3 und 4 lassen erkennen, dass die Deckelwand 6 einen zumindest geringfügig größeren Innendurchmesser als der Außendurchmesser der Schalenwand 4 hat. Dadurch ist gewährleistet, dass sich der Deckel 2 mit seiner Deckelwand 6 über die Schalenwand 4 der Schale 1 stülpen lässt, um nämlich die Schale 1 zu schließen. Letztendlich geht es darum, dass die beiden Teile - Schale und 1 und Deckel 2 - gemeinsam einen Raum bilden, wobei das eine Teil über das andere bzw. in das andere greift.

Erfindungsgemäß sind der Schale 1 und dem Deckel 2 Eingriffsmittel 7, 8 zugeordnet, die bei gegenseitigem Eingriff ein unbeabsichtigtes Lösen von Schale 1 und Deckel 2 verhindern.

Die Fig. lassen des Weiteren erkennen, dass die dem Deckel 2 zugeordneten Eingriffsmittel 8 im Sinne von Rastnasen 9 ausgebildet sind, die am freien Rand der Deckelwand 6 orthogonal von der Deckelwand 6 abragen. Entlang des Umfanges der Deckelwand 6 sind - bei dem hier gewählten Ausführungsbeispiel - insgesamt drei Rastnasen 9 vorgesehen, wobei mindestens zwei Rastnasen 9 erforderlich sind. In Bezug auf die Rastnasen 9 ist es durchaus denkbar, mehr als drei Rastnasen 9 vorzusehen, um nämlich eine erhöhte Stabilität der Anordnung zu realisieren.

Entsprechend der Ausgestaltung der Rastnasen 9 an der Deckelwand 6 ist die Schale 1 ausgestaltet, weist nämlich die Schale 1 komplementär ausgebildete Flansche 10 auf, die klammerartig ausgebildet sind. Die Flanschen 10 erstrecken sich von einem Randbereich 11 des Schalenbodens aus orthogonal und haben eine für das Halten bzw. die Klemmwirkung verantwortliche Schulter 12. Die Rastnase 9 des Deckels 2 lässt sich von beiden Seiten eines Flansches 10 her in den Bereich unter die Schulter 12 einschieben, wobei dort - jeweils rechts und links - unterschiedlich ausgebildete Keilflächen oder sonstige Erhebungen vorgesehen sind. Diese konstruktive Maßnahme führt dazu, dass sich je nach Einschieben von rechts oder von links her ein zumindest geringfügig beabstandetes oder dichtes Anbringen des Deckels 2 generieren lässt, wodurch innerhalb des Behältnisses eine offene (vgl. Fig. 3) oder geschlossene (vgl. Fig. 4) Situation schaffbar ist.

Die Fig. zeigen des Weiteren, dass die Schalenwand 4 auf deren Außenseite Abstandhalter 13 aufweist, die entlang des Umfanges der Schalenwand 4 ausgebildet sind. Diese Abstandhalter 13 schaffen eine Art Ringspalt zwischen der Innenseite der Deckelwand 6 und der Außenseite der Schalenwand 4 (vgl. Fig. 3 und 4), so dass bei nicht ganz anliegendem Deckel 2 auf dem Randbereich 11 eine zumindest geringfügige Belüftung bzw. Hinterlüftung realisierbar ist. Liegt der freie Rand der Deckelwand 6 komplett am Randbereich 11 an, wird nämlich durch die Eingriffsmittel 7, 8 eine Klemmwirkung hervorgerufen, ist eine geschlossene Situation innerhalb des Behältnisses geschaffen (Fig. 4).

Des Weiteren zeigen die Fig., dass die Schale 1 eine integrale Basis 14 in Form einer kreisringförmigen Wand aufweist, die vom Randbereich 11 aus, auf der der Schalenwand 4 abgewanden Seite des Schalenbodens 3, abragt. Die Basis 14 dient einerseits zum sicheren Positionieren und andererseits zum Stapeln des Behältnisses.

Schließlich sei angemerkt, dass das voranstehend erörterte Ausführungsbeispiel nebst Fig. lediglich der beispielhaften Erörterung der beanspruchten Lehre dient, dies jedoch nicht auf die Ausführungsbeispiele einschränken.

## Patentansprüche

1. Behältnis zur Aufnahme von Nährmedien, insbesondere zur Kultivierung von Mikroorganismen, Zellkulturen, Bakterien, mit einer Schale (1) und einem die Schale (1) schließenden Deckel (2), wobei die Schale (1) einen in Form einer Kreisfläche ausgebildeten Schalenboden (3) und eine vom Schalenboden (3) abragende, kreisringförmige Schalenwand (4) umfasst und wobei der Deckel (2) einen in Form einer Kreisfläche ausgebildeten Deckelboden (5) und eine vom Deckelboden (5) abragende, kreisringförmige Deckelwand (6) umfasst, wobei die Deckelwand (6) einen größeren Innendurchmesser als der Außendurchmesser der Schalenwand (4) hat oder die Schalenwand (4) einen größeren Innendurchmesser als der Außendurchmesser der Deckelwand (6) hat, so dass zum Schließen der Schale (1) die Deckelwand (6) über die Schalenwand (4) oder die Schalenwand (4) über die Deckelwand (6) stülpbar ist, wobei der Schale (1) und dem Deckel (2) Eingriffsmittel (7, 8) zugeordnet sind, die bei gegenseitigem Eingriff ein unbeabsichtigtes Lösen von Schale (1) und Deckel (2) verhindern und wobei die Eingriffsmittel (7) der Schale (1) klammerartig oder flanschartig zum Um- oder Eingreifen der Eingriffsmittel (8) des Deckels (2) ausgeführt sind und wobei sich die Flansche (10) von einem Randbereich (11) des Schalenbodens aus orthogonal erstrecken und eine für das Halten oder die Klemmwirkung verantwortliche Schulter (12) aufweisen, **dadurch gekennzeichnet, dass** die Eingriffsmittel (7) der Schale (1), in Umfangsrichtung der Schale (1) gesehen hälftig von beiden Seiten her, unterschiedlich hohe Keilflächen oder Erhöhungen haben, so dass die Eingriffsmittel (8) des Deckels (2) in Umfangsrichtung des Deckels (2) gesehen von beiden Seiten her mit unterschiedlicher Klemm- und Dichtwirkung in die Eingriffsmittel (7) der Schale (1), nämlich in den Bereich unter die Schulter (12), einschiebbar sind.

2. Behältnis nach Anspruch 1, **dadurch gekennzeichnet, dass** die Eingriffsmittel (7, 8) integrale Bestandteile der Schale (1) und des Deckels (2) sind.

3. Behältnis nach Anspruch 1 oder 2, **dadurch gekennzeichnet, dass** die Eingriffsmittel (7, 8) der Deckelwand (6) und der Schalenwand (4) zugeordnet sind.

4. Behältnis nach Anspruch 1 oder 2, **dadurch gekennzeichnet, dass** die Eingriffsmittel (7, 8) der Deckelwand (6) und einem umlaufenden äußeren Randbereich (11) der Schale (1) zugeordnet sind, wobei der äußere Randbereich (11) der Schale (1) zur Anlage für die Deckelwand (6) dient.

5. Behältnis nach Anspruch 4, **dadurch gekennzeichnet, dass** die Schalenwand (4) vom äußeren Randbereich (11) nach innen versetzt ausgebildet ist und dass der außerhalb der Schalenwand (4) liegende Randbereich (11), vorzugsweise ganz außen, die Eingriffsmittel (7) trägt.

6. Behältnis nach einem der Ansprüche 1 bis 5, **dadurch gekennzeichnet, dass** wobei die Eingriffsmittel (8) des Deckels (2) mindestens eine Rastposition umfassen können.

7. Behältnis nach einem der Ansprüche 1 bis 6, **dadurch gekennzeichnet, dass** die Eingriffsmittel (7) der Schale (1) mindestens eine Keilfläche oder Erhöhung zum klemmenden Eingriff mit den Eingriffsmitteln (8) des Deckels (2) haben.

8. Behältnis nach einem der Ansprüche 1 bis 7, **dadurch gekennzeichnet, dass** die Eingriffsmittel (7) der Schale (1) derart ausgebildet sind, dass die Eingriffsmittel (8) des Deckels (2) durch Drehen des Deckels (2) gegenüber der Schale (1) in Eingriff bringbar sind.

9. Behältnis nach einem der Ansprüche 1 bis 8, **dadurch gekennzeichnet, dass** der beidseitige Eingriff derart ausgelegt ist, dass von einer Seite her ein festes und/oder abdichtendes Verschließen und von der anderen Seite her ein Verschließen mit einem definierten Schlitz oder Spalt zum Gasaustausch möglich ist.

10. Behältnis nach einem der Ansprüche 1 bis 9, **dadurch gekennzeichnet, dass** der Schale (1) und dem Deckel (2) mindestens zwei, vorzugsweise drei äquidistant angeordnete Eingriffsmittel (7, 8) zugeordnet sind.

11. Behältnis nach einem der Ansprüche 1 bis 15, **dadurch gekennzeichnet, dass** die Schalenwand (4) auf der Außenseite und/oder die Deckelwand (6) auf der Innenseite Abstandhalter zur Beabstandung zwischen Deckelwand (6) und Schalenwand (4) hat, wobei die Abstandhalter (13) als sich vom Boden orthogonal erstreckende Stege ausgeführt sind und/oder wobei gegenüber eines Eingriffsmittels (7, 8) ein Abstandhalter (13) ausgebildet sein kann.

12. Behältnis nach Anspruch 11, **dadurch gekennzeichnet, dass** die Abstandhalter (13) vorzugsweise äquidistant in der Schalenwand (4) und/oder in der Deckelwand (6) ausgebildet sind.

13. Behältnis nach einem der Ansprüche 1 bis 12, **dadurch gekennzeichnet, dass** auf der der Schalenwand (4) abgewandten Seite des Schalenbodens (3) eine vorzugsweise am äußeren Ende des Randbereichs (11) in entgegen gesetzte Richtung abragende Basis (14) in Form einer kreisringförmigen Wand ausgebildet ist, die zum Positionieren der Schale (1) dient.

14. Behältnis nach einem der Ansprüche 1 bis 13, **dadurch gekennzeichnet, dass** die Schale (1) und/oder der Deckel (2) mit einer vorzugsweise maschinenlesbaren Codierung versehen sind.

15. Behältnis nach einem der Ansprüche 1 bis 14, **dadurch gekennzeichnet, dass** die Schale (1) und der Deckel (2) aus vorzugsweise durchsichtigem Kunststoff hergestellt sind, wobei die Schale (1) und der Deckel (2) spritzgusstechnisch hergestellt sind.

## Claims

1. Container for holding culture media, in particular for culturing microorganisms, cell cultures and bacteria, comprising a dish (1) and a lid (2) which closes the dish (1), the dish (1) comprising a dish bottom (3) in the form of a circular surface and an annular dish wall (4) projecting from the dish bottom (3), and the lid (2) comprising a lid bottom (5) in the form of a circular surface and an annular lid wall (6) projecting from the lid bottom (5), the lid wall (6) having a larger internal diameter than the external diameter of the dish wall (4) or the dish wall (4) having a larger internal diameter than the external diameter of the lid wall (6), such that, in order to close the dish (1), the lid wall (6) can be slid over the dish wall (4) or the dish wall (4) can be slid over the lid wall (6), engaging means (7, 8) being associated with the dish (1) and the lid (2), which means prevent unintended detaching of the dish (1) and lid (2) when mutually engaged, and the engaging means (7) of the dish (1) being clamp-like or flange-like in order to surround or engage with the engaging means (8) of the lid (2), and the flanges (10) extending orthogonally from a rim region (11) of the dish bottom and having a shoulder (12) which is responsible for the holding or clamping action, **characterised in that**, when viewed equally from both sides in the circumferential direction of the dish (1), the engaging means (7) of the dish (1) have wedge surfaces or elevated regions of different heights, such that the engaging means (8) of the lid (2), viewed in the circumferential direction of the lid (2) from both sides, can be inserted into the engaging means (7) of the dish (1), specifically into the region under the shoulder (12), so as to have different clamping and sealing actions.

2. Container according to claim 1, **characterised in that** the engaging means (7, 8) are integral components of the dish (1) and the lid (2).

3. Container according to either claim 1 or claim 2, **characterised in that** the engaging means (7, 8) are associated with the lid wall (6) and the dish wall (4).

4. Container according to either claim 1 or claim 2, **characterised in that** the engaging means (7, 8) are associated with the lid wall (6) and a circumferential outer rim region (11) of the dish (1), the outer rim region (11) of the dish (1) being intended to support the lid wall (6).

5. Container according to claim 4, **characterised in that** the dish wall (4) is offset inwardly from the outer rim region (11) and **in that** the rim region (11) located outside the dish wall (4), preferably completely outside, carries the engaging means (7).

6. Container according to any one of claims 1 to 5, **characterised in that** the engaging means (8) of the lid (2) can comprise at least one latching position.

7. Container according to any one of claims 1 to 6, **characterised in that** the engaging means (7) of the dish (1) have at least one wedge surface or elevated region for clamping engagement with the engaging means (8) of the lid (2).

8. Container according to any one of claims 1 to 7, **characterised in that** the engaging means (7) of the dish (1) are designed such that the engaging means (8) of the lid (2) can be brought into engagement by turning the lid (2) with respect to the dish (1).

9. Container according to any one of claims 1 to 8, **characterised in that** the mutual engagement is configured such that, from one side, a tight and/or sealing closure is possible and, from the other side, closure with a defined slot or gap for gas exchange is possible.

10. Container according to any one of claims 1 to 9, **characterised in that** at least two, preferably three, equidistantly arranged engaging means (7, 8) are associated with the dish (1) and the lid (2).

11. Container according to any one of claims 1 to 15, **characterised in that** the dish wall (4) has spacers on the outside and/or the lid wall (6) has spacers on the inside to form a spacing between the lid wall (6) and dish wall (4), the spacers (13) being designed as projections extending orthogonally from the bottom and/or it being possible for a spacer (13) to be formed opposite an engaging means (7, 8).

12. Container according to claim 11, **characterised in that** the spacers (13) are preferably formed so as to be equidistant in the dish wall (4) and/or in the lid wall (6).

13. Container according to any one of claims 1 to 12, **characterised in that** a base (14) is formed on the side of the dish bottom (3) facing away from the dish wall (4), which base preferably projects in the opposite direction from the outer end of the rim region (11), is in the form of an annular wall and is intended for positioning the dish (1).

14. Container according to any one of claims 1 to 13, **characterised in that** the dish (1) and/or the lid (2) are provided with a preferably machine-readable coding.

15. Container according to any one of claims 1 to 14, **characterised in that** the dish (1) and the lid (2) are made of preferably transparent plastics material, the dish (1) and the lid (2) being produced by injection moulding.

## Revendications

1. Contenant destiné à la réception de milieux nutritifs, en particulier à la culture de microorganismes, de cultures cellulaires, de bactéries, avec une coupelle (1) et un couvercle (2) fermant la coupelle (1), dans lequel la coupelle (1) comprend un fond de coupelle (3) constitué en forme de surface circulaire et une paroi de coupelle (4) en forme d'anneau de cercle s'élevant à partir du fond de coupelle (3), et dans lequel le couvercle (2) comprend un fond de couvercle (5) constitué en forme de surface circulaire et une paroi de couvercle (6) en forme d'anneau de cercle s'élevant à partir du fond de couvercle (5), dans lequel la paroi de couvercle (6) a un diamètre intérieur plus grand que le diamètre extérieur de la paroi de coupelle (4), ou la paroi de coupelle (4) a un diamètre intérieur plus grand que le diamètre extérieur de la paroi de couvercle (6) de telle sorte que, pour fermer la coupelle (1), la paroi de couvercle (6) peut venir coiffer la paroi de coupelle (4), ou la paroi de coupelle (4) peut venir coiffer la paroi de couvercle (6), dans lequel des moyens d'engagement (7, 8) sont affectés à la coupelle (1) et au couvercle (2) et qui, quand ils sont engagés mutuellement, empêchent un desserrage inopiné de la coupelle (1) et du couvercle (2), et dans lequel les moyens d'engagement (7) de la coupelle (1) sont réalisés en forme d'agrafe ou de bride pour l'enserrement ou l'engagement des moyens d'engagement (8) du couvercle (2), et dans lequel les brides (10) s'étendent à angle droit à partir d'une zone de bord (11) du fond de coupelle et comportent un épaulement (12) responsable de la retenue ou de l'action de serrage, **caractérisé en ce que** les moyens d'engagement (7) de la coupelle (1), vus dans la direction circonférentielle du de la coupelle (1), ont, à moitié à partir des deux côtés, des surfaces de coin ou reliefs de différentes hauteurs de telle sorte que les moyens d'engagement (8) du couvercle (2), vus dans la direction circonférentielle du couvercle (2), à partir des deux côtés, peuvent être introduits avec une action de serrage et d'étanchéité différente dans les moyens d'engagement (7) de la coupelle (1), à savoir dans la zone sous l'épaulement (12).

2. Contenant selon la revendication 1, **caractérisé en ce que** les moyens d'engagement (7, 8) font partie intégrante de la coupelle (1) et du couvercle (2).

3. Contenant selon la revendication 1 ou 2, **caractérisé en ce que** les moyens d'engagement (7, 8) sont affectés à la paroi de couvercle (6) et à la paroi de coupelle (4).

4. Contenant selon la revendication 1 ou 2, **caractérisé en ce que** les moyens d'engagement (7, 8) sont affectés à la paroi de couvercle (6) et à une zone de bord (11) extérieure périphérique de la coupelle (1), dans lequel la zone de bord (11) extérieure de la coupelle (1) sert d'appui à la paroi de couvercle (6).

5. Contenant selon la revendication 4, **caractérisé en ce que** la paroi de coupelle (4) est constituée de façon décalée vers l'intérieur à partir de la zone de bord (11) extérieure, et **en ce que** la zone de bord (11) située à l'extérieur de la paroi de coupelle (4), de préférence totalement extérieurement, porte les moyens d'engagement (7).

6. Contenant selon l'une des revendications 1 à 5, **caractérisé en ce que** les moyens d'engagement (8) du couvercle (2) peuvent comprendre au moins une position d'encliquetage.

7. Contenant selon l'une des revendications 1 à 6, **caractérisé en ce que** les moyens d'engagement (7) de la coupelle (1) ont au moins une surface de coin ou un relief pour l'engagement bloquant avec les moyens d'engagement (8) du couvercle (2).

8. Contenant selon l'une des revendications 1 à 7, **caractérisé en ce que** les moyens d'engagement (7) de la coupelle (1) sont constitués de telle sorte que les moyens d'engagement (8) du couvercle (2) peuvent être mis en prise par la rotation du couvercle (2) par rapport à la coupelle (1).

9. Contenant selon l'une des revendications 1 à 8, **caractérisé en ce que** l'engagement des deux côtés est conçu de telle sorte que, à partir d'un côté, une obturation fixe et/ou étanche et, à partir de l'autre côté, une obturation avec une fente ou un interstice défini(e) pour l'échange gazeux est possible.

10. Contenant selon l'une des revendications 1 à 9, **caractérisé en ce qu'**au moins deux, de préférence trois, moyens d'engagement (7, 8) disposés de façon équidistante sont affectés à la coupelle (1) et au couvercle (2).

11. Contenant selon l'une des revendications 1 à 15, **caractérisé en ce que** la paroi de coupelle (4), sur le côté extérieur, et/ou la paroi de couvercle (6), sur le côté intérieur, a des pièces d'espacement destinées à l'espacement entre la paroi de couvercle (6) et la paroi de coupelle (4), dans lequel les pièces d'espacement (13) sont réalisée en tant qu'entretoises s'étendant à angle droit par rapport au fond et/ou dans lequel une pièce d'espacement (13) peut être constituée en face d'un moyen d'engagement (7, 8).

12. Contenant selon la revendication 11, **caractérisé en ce que** les pièces d'espacement (13) sont constituées de préférence de façon équidistante dans la paroi de coupelle (4) et/ou dans la paroi de couvercle (6).

13. Contenant selon l'une des revendications 1 à 12, **caractérisé en ce que**, sur le côté du fond de coupelle (3) éloigné de la paroi de coupelle (4), il est constitué une base (14) dépassant de préférence à l'extrémité extérieure de la zone de bord (11) en sens opposé, sous forme d'une paroi en forme d'anneau de cercle, qui sert au positionnement de la coupelle (1).

14. Contenant selon l'une des revendications 1 à 13, **caractérisé en ce que** la coupelle (1) et/ou le couvercle (2) sont munis d'un codage de préférence lisible par machine.

15. Contenant selon l'une des revendications 1 à 14, **caractérisé en ce que** la coupelle (1) et le couvercle (2) sont formés d'une matière plastique de préférence transparente, dans lequel la coupelle (1) et le couvercle (2) sont fabriqués par moulage par injection.
